(19) Europäisches Patentamt
European Patent Office
Office européen des brevets

(11) **EP 4 535 233 A1**

(12) **EUROPEAN PATENT APPLICATION**

(43) Date of publication:
**09.04.2025 Bulletin 2025/15**

(21) Application number: **23306730.5**

(22) Date of filing: **06.10.2023**

(51) International Patent Classification (IPC):
**G06N 3/045** (2023.01)    **G16H 50/20** (2018.01)

(52) Cooperative Patent Classification (CPC):
**G16H 50/20; G06N 3/045; G16H 30/40;
G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Université de Bretagne Occidentale
29200 Brest (FR)**
• **IMT Atlantique Bretagne Pays de la Loire
29238 Brest cedex 03 (FR)**
• **Institut National de la Santé et de la Recherche
Médicale
75013 Paris (FR)**

(72) Inventors:
• **ZEGHLACHE, Rachid
29200 Brest (FR)**
• **CONZE, Pierre-Henri
29200 Brest (FR)**
• **EL HABIB DAHO, Mostafa
29200 Brest (FR)**
• **LAMARD, Mathieu
29820 Guilers (FR)**
• **QUELLEC, Gwenolé
29200 Brest (FR)**

(74) Representative: **Novagraaf Technologies
Bâtiment O2
2, rue Sarah Bernhardt
CS90017
92665 Asnières-sur-Seine Cedex (FR)**

(54) **METHOD AND APPARATUS FOR PREDICTING PROGRESSION OF A PATHOLOGY**

(57)    A method and apparatus are proposed for training a model for predicting information representative of a pathology progression at a future time $(t+\Delta t)$ from one or more examination images (for instance one image 2D + a 3D volume) of a region of interest of a patient captured at a current time (t), said method comprising:
- getting (20) a training dataset, comprising time series of examination images of said region of interest from patients associated with labels, said labels (S(t)) comprising said information representative of a pathology progression,
- training (E1, 21, 22) the machine learning model (MLM) with inputs from said training dataset comprising, at least one of said inputs comprising a pair of consecutive examination images comprising a first image (x(ti)) associated with a first time (ti) and first label (S(ti)), a second image (x(ti+1)) associated with a second time (ti+1) and a second label (S(ti+1)), to output a target intermediate label ($TS(t_{mix})$) at an intermediate time selected within a time interval ([ti,ti+1]) between the first and second examination images, said target intermediate label ($TS(t_{mix})$) being determined for said intermediate time ($t_{mix}$), based on a given pathology progression profile (I(t))

**FIG. 2**

## Description

### Technical Field

The invention relates to the field of processing of examination images of an area of a patient, in particular to prediction of progression of a pathology at a future time based on examination images captured at a current time.
It applies, for instance, but not only, to prediction of progression of Diabetic Retinopathy (DR).

### Background

According to the International Diabetes Federation, by 2045, diabetes will impact 700 million individuals globally, with over one-third suffering from Diabetic Retinopathy (DR). DR is the leading cause of vision loss worldwide and is caused by high blood sugar damaging retinal vessels, leading to swelling and leakage. Color fundus photographs (CFP) are used clinically to detect DR.
Severity is graded in five classes (0, 1, 2, 3 and 4) using the International Clinical DR (ICDR) scale: 0 is no apparent DR, 1 is mild non-proliferative DR (NPDR), 2 is moderate NPDR, 3 is severe NPDR, and 4 is proliferative DR (PDR). Early detection and treatment, particularly in mild to moderate NPDR, may slow DR progression and reduce blindness incidence.
Document by Anegondi et al., entitled "Deep Learning to Predict Geographic Atrophy Area and Growth Rate from Multimodal Imaging", published by the American Academy of Ophtalmology, in 2022, discloses training and inferring an artificial intelligence model to predict a geographic atrophy lesion area in 3D Optical Coherence Tomography image scans of a portion of an eye of a patient, at a current time (corresponding to the time of capture of the 3D images) and also its growth rate at a given subsequent time, for instance at the end of a predetermined period of time, for instance of two years. The model is trained based on a database comprising 3D OCT image scans, associated each with one or more labels indicating how and whether the geographic atrophy lesion of the patient progressed over this predetermined period of time, for example a size or volume growth of the GA lesion.
An inconvenient of this solution is that that it is only able to predict an information representative of a progression of the pathology at the end of this predetermined period of time. In order to get a prediction at another subsequent time, another specific training of the machine learning model needs to be carried out.
However, it would be very helpful to a practitioner to get an information about a progression of the pathology at any future time within a given time period, for example to schedule a next examination more efficiently or to adjust a treatment of the pathology.
There is thus a need for method and apparatus for training and using a model to predict evolution of a pathology, which is more flexible and allows to get an information representative of a progression of a pathology at any time within a given period of time.

### Summary

The scope of protection is set out in the independent claims. The embodiments, examples and features, if any, described in this specification that do not fall under the scope of the protection are to be interpreted as examples useful for understanding the various embodiments or examples that fall under the scope of protection.
According to a first aspect, it is proposed a method for training a machine learning model for predicting information representative of a pathology progression at a future time from one or more examination images ($x(t)$) of a region of interest of a patient captured at a current time, said method comprising:

- getting a training dataset, comprising time series of consecutive examination images of said region of interest from patients associated with labels, said labels comprising said information representative of a pathology progression,
- training the machine learning model with inputs from said training dataset comprising, at least one of said inputs comprising a pair of consecutive examination images comprising a first image associated with a first time and a first label, a second image associated with a second time and a second label, to output a target intermediate label at an intermediate time selected within a time interval between the first and second examination images, said target intermediate label being determined for said intermediate time, based on a given pathology progression profile.

It is thus proposed a method for training a predictive model that is based on input pairs of consecutive examination images of patients extracted from a "longitudinal" training dataset comprising consecutive examination images of patients and associated labels.
In one or more examples, interval of time between both consecutive between 1 year and 5 years. On average, the interval of time is around 2.5 years, with a standard deviation of 2.2 years.

Use of these input pairs of consecutive examination images combined with intermediate or soft labels (that modulate the learnt decision boundaries), provides the model with more information and allows the machine learning model to learn information about pathology progression embedded within textures of examination images.

Selection of intermediate times within the time interval between both consecutive examination images allows the machine learning model to learn to predict a value of an information representative of the pathology progression at any time within this time interval.

Examination images may be acquired by any imaging modality, in 2D or 3D, or a combination of imaging modalities.

In one or more examples, examination images are color fundus photographs (CFP) and the method is applied to prediction of progression of diabetic retinopathy (DR). In this case, information representative of pathology progression comprises a severity grade. As an alternative, they may comprise an indicator of growth, for example of volume growth or size growth.

More than one pathology progression profiles may be used (linear, non-linear) for training the machine learning model. Different versions of the trained machine learning model may be obtained corresponding to these different profiles.

This embodiment may comprise other features, alone or in combination, that are presented hereinafter.

According to one or more exemplary embodiments, said machine learning model comprises a first neural network and a second neural network, said training comprises pre-training at least the first neural network of the machine learning model with said input data from said training dataset to output a first vector of features representative of the pathology progression on a time interval between the first time associated with a first image of the input pair and the intermediate time, and said training further comprises training the machine learning by using said first vector of features as input to the second neural network.

The first neural network is pre-trained to perform a longitudinal pretext task, which allows to encode in the first vector of features, a latent representation of texture information within the time interval between the consecutive images of the input pair.

In one or more examples, the full machine learning model is trained once the first neural network is pre-trained. However, pre-training and training may also be performed in parallel.

According to one or more exemplary embodiments, said pre-training comprises selecting said intermediate time by mixing-up the first and second times of the consecutive examination images of the input pair, by linear interpolation, according to a given distribution function.

Time or longitudinal mix up allows to select intermediate time randomly according to a distribution law. This performs data augmentation of the longitudinal dataset and allows to train the neural network on a larger number of different intervals of times within ti and ti+1. Another advantage is to avoid overfitting.

According to one or more exemplary embodiments, the pre-training further comprises determining an intermediate image at the selected intermediate time by performing linear interpolation between the first and second images of the input pair.

The first neural network is thus trained on the intermediate image corresponding to the input intermediate time.

According to one or more exemplary embodiments, the first neural network comprising a plurality of connected layers, said pre-training further comprises mixing up a first batch of internal features of at least one of said layers associated with the first image with a second batch of internal features of at least one of said layers associated with the second image.

This is based on a technique called "manifold mix up" which has been extended therein to time mix up. Mixing the internal features of a layer of the first neural network instead of determining the intermediate image, allows the neural network to take time into account more efficiently and thus to provide more informative hidden representation of the pathology progression.

According to one or more exemplary embodiments, said pre-training further comprises:

- obtaining a predicted information of pathology progression by presenting said first vector of features to a third neural network, configured to be trained simultaneously with the first neural network to output the predicted information of pathology progression,
- determining at least one error between said predicted information of pathology progression and said target information of pathology progression,
- backpropagating said error to the first neural network while at least one stopping criterion based on at least said error has not been reached.

In one or more examples, prediction of a value of information representative of the pathology progression from the first vector of features is performed by a third neural network, also called "classification head", which is simultaneously trained with the first neural network.

In one or more examples, the stopping criterion is the first error being less than or equal to a first threshold.

According to one or more exemplary embodiments, the pre-training of the at least one first neural network comprises:

- determining, based on a second loss function, a second error between the intermediate time and a predicted intermediate time determined by regression from the output first vector of features, and
- backpropagating the second error while at least one stopping criterion based on at least said second error has not been reached.

This allows to ensure time consistency of the model and thus to enhance the quality of features extraction. A total loss comprising a sum of both losses may be used.

In one or more examples, this estimation is performed by a fourth neural network, also called "regression head", which is simultaneously trained with the first neural network and possibly the third neural network. In one or more examples, this fourth neural network also comprises a feed forward artificial neural network, of Multi-Layer Perceptron, MLP, type, comprising two of more layers.

Both third and fourth neural networks are only used for pre-training. They are not comprised in the final model.

According to one or more exemplary embodiments, the training comprises:

- getting second input data from said training dataset, at least one of said input data comprising a first image of a pair of consecutive examination images from the training dataset, and a first associated label,
- selecting an intermediate time between the first time and a second time of a second image of the pair, by linear interpolation, according to a given distribution function, and
- presenting said second input data to the first neural network and getting a first vector of features associated with said first time as output,
- presenting said first vector of features and said intermediate time as input to the second neural network and obtaining a second vector of features as output, said second vector of features having a same size as the first vector of features,
- determining a target intermediate label associated with said intermediate time based on said given pathology progression profile,
- predicting an output intermediate label, said output intermediate label being obtained by regression from the second vector of features, and
- backpropagating a third error based at least on a third function loss between the output intermediate label and the target intermediate label, while a least one stopping criterion based at least on this third error has not been reached.

According to one or more exemplary embodiments, the second neural network comprises a Neural Ordinary Differential Equation, NODE, network, that is trained to output the second vector of features by solving a differential equation parametrized by the intermediate time.

This new type of neural networks is configured to parametrize the continuous dynamics of ordinary differential equations. It is ideally suited for solving time related problems.

In one or more examples the NODE network is a combination of dense layers followed by a tanh activation function.

It should be noted that the method is not limited to NODES and any other time-aware neural network may be used in place of a NODE.

Correlatively, according to a second aspect, an apparatus for training a machine learning module for predicting an information representative of a pathology progression at a future time from one or more examination images of a region of interest of a patient captured at a current time, comprises means for:

- getting a training dataset, comprising time series of consecutive examination images of said region of interest from patients associated with labels, said labels comprising said information representative of a pathology progression,
- training the machine learning model with inputs from said training dataset comprising, at least one of said inputs comprising a pair of consecutive examination images comprising a first image associated with a first time and first label, a second image associated with a second time and a second label, to output a target intermediate label at an intermediate time selected within a time interval between the first and second examination images, said target intermediate label being determined for said intermediate time, based on a given pathology progression profile.

According to one or more exemplary embodiments, the apparatus may comprise means for performing one or more or all steps of the method according to the first aspect. The means may include circuitry configured to perform one or more or all steps of a method according to the first aspect.

According to one or more exemplary embodiments, the apparatus comprises at least one processor and at least one memory storing instructions that, when executed by the at least one processor, cause the apparatus to perform:

- getting a training dataset, comprising time series of consecutive examination images of said region of interest from patients associated with labels, said labels comprising said information representative of a pathology progression,
- training the machine learning model with inputs from said training dataset comprising, at least one of said inputs comprising a pair of consecutive examination images comprising a first image associated with a first time and first label, a second image associated with a second time and a second label, to output a target intermediate label at an intermediate time selected within a time interval between the first and second examination images, said target intermediate label being determined for said intermediate time, based on a given pathology progression profile.

Correlatively, according to a third aspect, a method for predicting a severity grade representative of a pathology progression at a future time from one or more examination images of a region of interest of a patient captured at a current time, comprises:

- obtaining the one or more examination images at said current time and selecting the future time,
- presenting the one or more examination images and said future time as input to a trained machine learning model, said machine learning model having been trained with inputs from a training dataset comprising at least one of said inputs comprising a pair of consecutive examination images comprising a first image associated with a first time and first label, a second image associated with a second time and a second label, to output a target intermediate label at an intermediate time selected within a time interval ([ti,ti+1]) between the first and second examination images, said target intermediate label being determined for said intermediate time, based on a given pathology progression profile, and
- obtaining a predicted information of pathology progression for said future time as output.

As the machine learning model has been trained to predict an information representative of pathology progression based on a plurality of input pairs of consecutive examination images captured within various intervals of time, any future time within a period of time extending at least between 6 months and several years, for example 6 years, may be selected.

Correlatively, according to a fourth aspect, an apparatus for predicting an information representative of a pathology progression at a future time from one or more examination images of a region of interest of a patient captured at a current time, comprises means for:

- obtaining the one or more examination images at current time and selecting the future time,
- presenting the one or more examination images and said future time as input to a trained machine learning model, said machine learning model having been trained with inputs from a training dataset comprising at least one of said inputs comprising a pair of consecutive examination images comprising a first image associated with a first time and first label, a second image associated with a second time and a second label, to output a target intermediate label at an intermediate time selected within a time interval between the first and second examination images, said target intermediate label being determined for said intermediate time, based on a given pathology progression profile, and
- obtaining a predicted information of pathology progression for said future time as output.

The apparatus may comprise means for performing one or more or all steps of the method according to the third aspect. The means may include circuitry configured to perform one or more or all steps of a method according to the third aspect.

According to one or more exemplary embodiments, the apparatus comprises at least one processor and at least one memory storing instructions that, when executed by the at least one processor, cause the apparatus to perform:

- obtaining the one or more examination images at current time and selecting the future time,
- presenting the one or more examination images and said future time as input to a trained machine learning model, said machine learning model having been trained with inputs from a training dataset comprising at least one of said inputs comprising a pair of consecutive examination images comprising a first image associated with a first time and first label, a second image associated with a second time and a second label, to output a target intermediate label at an intermediate time selected within a time interval between the first and second examination images, said target intermediate label being determined for said intermediate time, based on a given pathology progression profile, and
- obtaining a predicted information of pathology progression for said future time as output.

Correlatively, according to a fifth aspect, a computer program comprises instructions which, when the program is executed by a computer, cause the computer to carry out the steps of the method of according to the first aspect or the method according to the third aspect.

According to a sixth aspect, a non-transitory computer-readable medium stores the computer program according to the fifth aspect.

Correlatively, according to another aspect, a system for processing examination images of a region of interest of a patient comprises an apparatus for predicting an information representative of a pathology progression at a future time from one or more examination images of a region of interest of a patient captured at a current time, according to the fourth aspect. In one or more examples, it may further comprises an apparatus for training a machine learning model according to the second aspect.

The apparatuses, the computer programs and the system may present the same advantages as the methods according to the first and the third aspects.

**Brief Description of the Figures**

Some embodiments are now described, by way of example only, and with reference to the accompanying drawings, in which:

- Figure 1 schematically illustrates an example of functional architecture of an apparatus and system for training a model to predict an information representative of an evolution of a pathology, according to one or more embodiments,
- Figure 2 is a flowchart schematically illustrating a method for training a model to predict an information representative of an evolution of a pathology according to one or more exemplary embodiments,
- Figure 3 schematically illustrates an example of an architecture of neural networks used for pre-training of a first neural network of the model according to one or more exemplary embodiments,
- Figure 4A is a flowchart schematically describing the pretraining of the first neural network according to an exemplary embodiment,
- Figure 4B is a flowchart schematically describing the pretraining of the first neural network according to another exemplary embodiment,
- Figure 5A schematically illustrates some examples of curves representing distribution laws that may be used for temporally mixing up examination images of a training dataset according to one or more exemplary embodiments,
- Figure 5B schematically illustrates some examples of curves representing pathology progression profiles that may be used for training the model according to one or more exemplary embodiments,
- Figure 6 schematically illustrates an example of an architecture of the machine learning model according to one or more embodiments,
- Figure 7 is a flowchart schematically describing the training of the machine learning model comprising the pre-trained first neural network according to one or more exemplary embodiments,
- Figure 8 schematically illustrates an example of functional architecture of a system for processing examination images comprising an apparatus for predicting an information representative of an evolution of a pathology, according to one or more embodiments,
- Figure 9 is a flowchart schematically illustrating a method for predicting an information representative of an evolution of a pathology using the trained machine learning model, according to one or more exemplary embodiments,
- Figures 10A-10C present tables comparing performances of the method for predicting an information representative of a pathology procession and known methods, in various experiments, according to one or more exemplary embodiments, and
- Figure 11 schematically illustrates an example of structural architecture of an apparatus for training a machine learning model to predict an information representative of an evolution of a pathology and an apparatus for predicting an information representative of an evolution of a pathology using the trained model, according to one or more exemplary embodiments.

**Description of Embodiments**

Detailed example embodiments are disclosed herein. However, specific structural and/or functional details disclosed herein are merely representative for purposes of describing example embodiments and providing a clear understanding of the underlying principles. However, these example embodiments may be practiced without these specific details. These example embodiments may be embodied in many alternate forms, with various modifications, and should not be construed as limited to only the embodiments set forth herein. In addition, the figures and descriptions may have been simplified to illustrate elements and / or aspects that are relevant for a clear understanding of the present invention, while eliminating, for purposes of clarity, many other elements that may be well known in the art or not relevant for the understanding of the invention.

In the following, different exemplary embodiments are described that are applied to train a machine learning model to predict an information representative of a pathology progression from time series of examination images of a given region of a plurality of patients and then to apply the trained model to predict at a future time $t+\Delta t$ a value of said information representation the pathology progression for a given patient from one examination image captured a current time t.

In examples described therein, focus is made on a specific pathology of the eye, namely Diabetic Retinopathy, and on examination images of a region comprising part of an eye of a patient, that are Colors Fundus Photographs (CFP). Progression of this pathology is represented by an information of severity, or severity grade, which is graded in five classed (0, 1, 2,3 and 4) using the International Clinical DR (ICDR) scale: 0 is no apparent DR, 1 is mild non-proliferative DR (NPDR), 2 is moderate NPDR, 3 is severe NPDR, and 4 is proliferative DR (PDR).

Of course, methods, apparatuses and system that are disclosed herein are not limited to this example of application. On the contrary, they also apply to any other pathology related to any other region of a patient based on any other type of 2D or 3D examination images captured by any other imaging modalities. Other examples of applications include prediction of progression of age-related macular degeneration using autofluorescence imaging or optical coherence tomography and prediction of progression of cognitive impairment due to Alzheimer's disease using magnetic resonance imaging.

Depending on the context, the predicted information representative of the pathology progression may be any other type of indicator, such as a rate of size or volume growth, or more generally any information that can be used to characterize or quantify disease progression.

Machine Learning (ML) provides computer systems with the ability to perform tasks, without explicitly being programmed, by making inferences based on patterns found in the analysis of data. Machine learning explores the study and construction of algorithms, also referred to herein as tools, that may learn from existing data and make predictions about new data. Such machine-learning algorithms operate by building an ML model from example training data in order to make data-driven predictions or decisions expressed as outputs or assessments. Although example embodiments are presented with respect to a few machine-learning tools, the principles presented herein may be applied to other machine-learning tools.

In example embodiments, a supervised mode is used for training ML and building the ML model. Supervised ML uses prior knowledge (e.g., examples that correlate inputs to outputs or outcomes) to learn the relationships between the inputs and the outputs. The goal of supervised ML is to learn a function that, given some training data, best approximates the relationship between the training inputs and outputs so that the ML model can implement the same relationships when given inputs to generate the corresponding outputs. Some examples of commonly used supervised-ML algorithms are Logistic Regression (LR), Naive-Bayes, Random Forest (RF), neural networks (NN), deep neural networks (DNN), convolutional neural networks (CNN), matrix factorization, and Support Vector Machines (SVM).

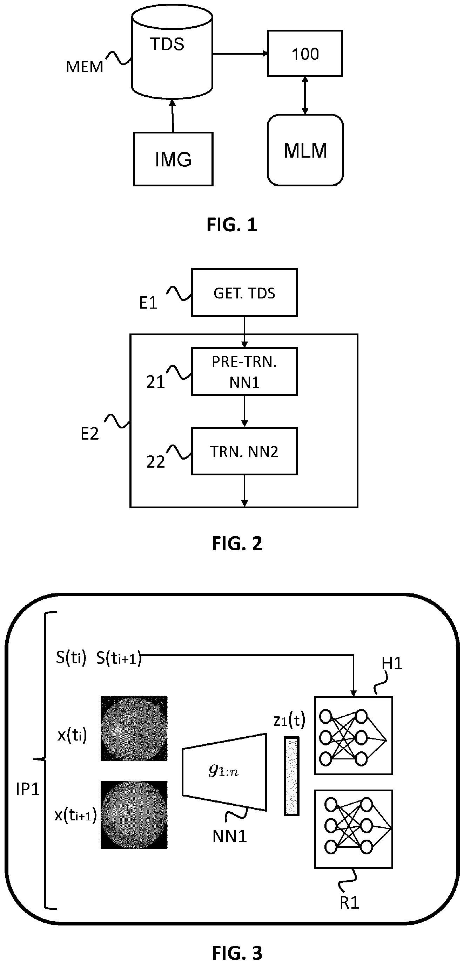

In relation with FIG. 1, an apparatus 100 for training a machine learning model MLM to predict an information representative of a progression of a pathology is presented, in an exemplary context in which it may be deployed. Said apparatus 100 comprises communication means with the machine learning model MLM, through which it is configured to transmit training data to the machine learning model MLM. Apparatus 100 further comprises communication means with a memory MEM, for example organized as a database, in which a training dataset TDS is stored.

The training dataset TDS comprises examples of values for the input features associated with labels indicating the features to be output. The machine-learning algorithms utilize the training data to find correlations among identified features that affect the outcome. A feature is an individual measurable property of a phenomenon being observed. The concept of a feature is related to that of an explanatory variable used in statistical techniques such as linear regression. Choosing informative, discriminating, and independent features is important for effective operation of ML in pattern recognition, classification, and regression. Features may be of different types, such as numeric features, strings, and graphs.

In one or more examples, the training dataset TDS comprises a plurality v of times series of examination images of the region of interest of a plurality of a plurality of patients. As it comprises several examination images of the region of interest of a same patient over time, it is also referred to as a longitudinal training dataset. In a time series, consecutive examination images were captured at time intervals ranging for example from 6 months to 6 years, on average around 2.5 years with a standard deviation of 2.2 years.

The examination images are stored in the training dataset TDS with an associated time of acquisition and with an annotation or label, for example made by a practitioner, of a value of the information representative of the pathology progression, here a severity grade. As this training dataset comprises time series of follow-up examination images of a same patient, it is also referred to a longitudinal training dataset.

In one or more examples, this training dataset TDS is a local dataset that may have been populated by follow-up examination images, here CFP images, captured by one or more imaging systems IMG that may be based on different imaging modalities, over a given period up to several years. The one or more imaging systems are for example deployed and in operation in a clinical environment, such as a hospital.

In one or more examples, the training dataset TDS comprises consecutive patient-specific image pairs for the collection of all CFP images.

As an alternative, the training dataset TDS is a distant dataset, for instant a public database, such as for example OPHDIAT for CFP images, as will be described hereinafter, in relation with FIG.S 10A-10C.

As will be detailed below, the machine learning model MLM comprises one or more neural networks that are trained to perform different functions and may be placed in series or in parallel.

In one or more examples, apparatus 100 comprising means for:

- getting a training dataset, comprising time series of (follow-up) examination images of said region of interest from patients associated with labels, said labels comprising said information representative of a pathology progression,
- training the machine learning model with inputs from said training dataset comprising, at least one of said inputs comprising a pair of consecutive examination images comprising a first image associated with a first time and first label, a second image associated with a second time and a second label, to output a target intermediate label at an intermediate time selected within a time interval between the first and second examination images, said target intermediate label being determined for said intermediate time, based on a given pathology progression profile.

In one or more examples, the apparatus 100 is configured to implement a method for training a machine learning model to predict an information representative of a pathology progression, that will be described hereinafter in relation with FIG. 2.

FIG. 2 shows a flowchart of a method for training a machine learning model for predicting information representative of a pathology progression at a future time, $t+\Delta t$, from one or more examination images of an area of a patient captured at a current time t. For example, this method is implemented by the apparatus 100 of FIG. 1. While the steps of the method are described in a sequential manner, the man skilled in the art will appreciate that some steps may be omitted, combined, performed in different order and / or in parallel.

In the following, it is assumed that the machine learning model MDL comprises a first neural network NN1 and a second neural network NN2, placed in series, that are each configured and trained to perform distinct tasks as will be described therein.

In a step E1, a longitudinal dataset, for instance the training dataset TDS described above in relation with FIG. 1.

In a step E2 the machine learning model MLM is trained. In one or more examples, said training step comprises a pre-training step 21, the first neural network NN1 of the model MLM is pre-trained with first input data IP1 extracted from said longitudinal dataset TDS. First input data IP1 comprise pairs of consecutive examination images. More precisely, such a pair comprises a first image $x(t_i)$ associated with time $t_i$, a second image $X(t_{i+1})$ associated with time $t_{i+1}$. For example, the training dataset TDS contains all pairs of consecutive examination images $(x(t_i), x(t_{i+1}))$ that are from a same patient where $x(t_i)$ is scanned before $x_{t_{i+1}}$ with $i \in [0, m - 2]$, m being the number of visits for a given eye. The first input data OP1 further comprise labels, namely severity grades $S(t_i)$ and $S(t_{i+1})$ respectively associated with the first and second images of the pair.

In one or more examples, other input pairs may be used comprising non consecutive examination images of a time-series of a patient, such as for example, the first and the fifth image of the time series.

The expected output comprises a first vector of features $z_1(t)$ whose features are learnt to be representative of the pathology progression on a time interval between respective times $t_i$ and $t_{i+1}$ associated with both examination images of input pairs. It is also referred to as a latent representation of the pathology progression, as it encodes information about the pathology progression that are embedded into the texture of the examination images of the input pair.

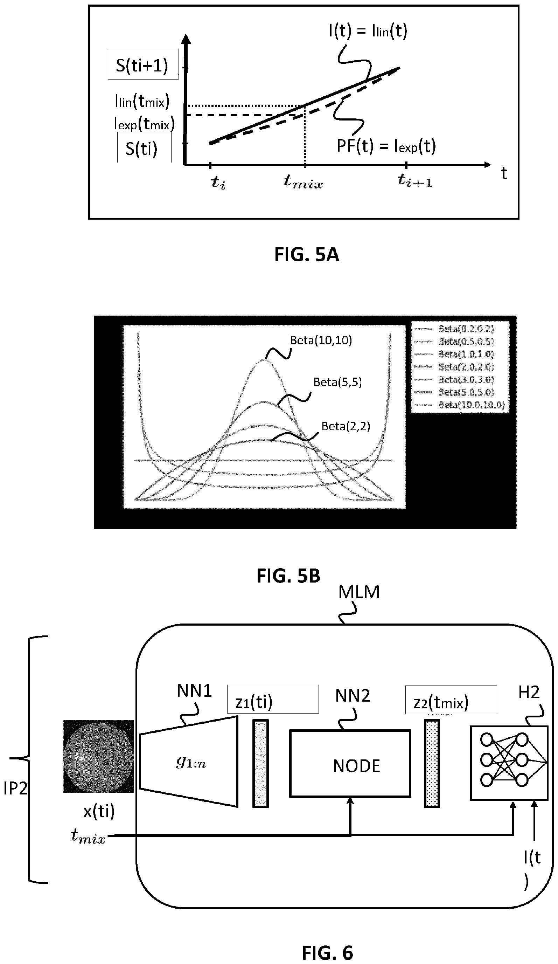

During pre-training, for each new input pair IP1, a severity grade S(t) is predicted from the output first vector $z_1(t)$ and at least one loss function I is used to determine an error between the predicted severity grade PS(t) and a target severity grade TS(t) derived from a given profile of pathology progression I(t) which is applied to the first and second times $t_i, t_{i+1}$. Based on the assumption that progression of DR pathology is a slow process, monotonic disease progression profiles comprising at least a linear profile $I_{lin}(t)$ and an exponential profile $I_{exp}(t)$, as will be illustrated by FIG. 5A. The linear profile $I_{lin}(t)$ may be expressed as:

$$I_{lin}(t) = \frac{t-t_i}{t_{i+1}-t_i}\left(S(t_{i+1}) - S(t_i)\right) + S(t_i) \qquad (1)$$

The exponential profile may be expressed as:

$$I_{exp}(t) = S(t_i)\left(\frac{S(t_{i+1})}{S(t_i)}\right)^{\frac{t-t_i}{t_{i+1}-t_i}} \qquad (2)$$

Preferably, a same progression profile I(t) is used for the whole training process. Different versions of the machine learning model may be thus obtained by training it with different profiles.

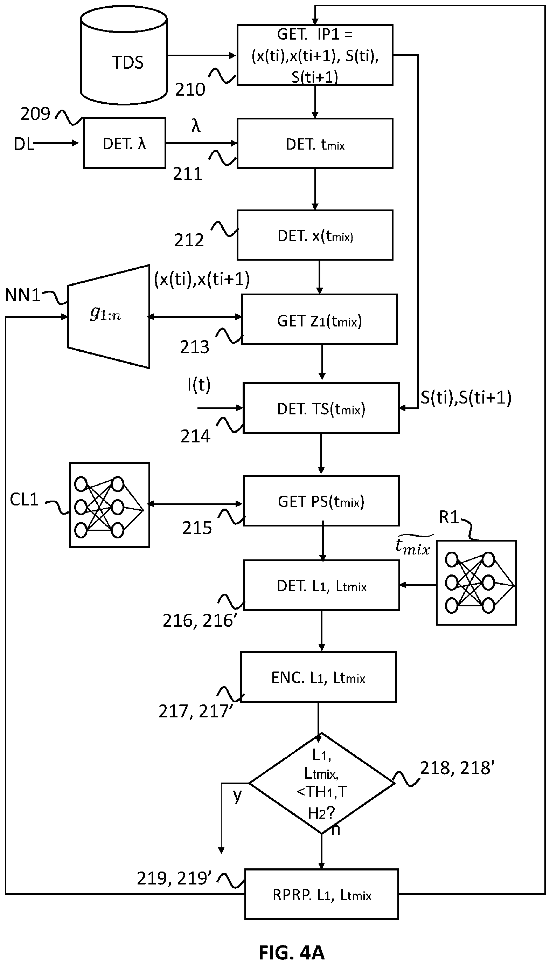

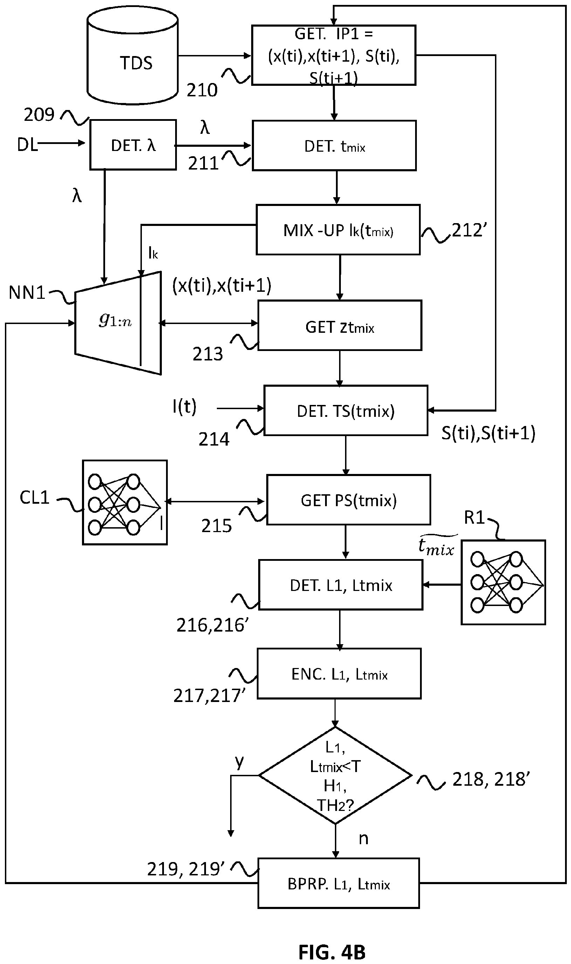

The error is further backpropagated to the first neural network and internal features or weights of the first neural network NN1 are updated accordingly. Pre-training is repeated with other input pairs IP1 from the training dataset TDS until a stopping criterion is reached, for example while the error is greater than a predetermined threshold. An exemplary embodiment of this pre-training step will be detailed hereinafter in relation with FIGs. 4A and 4B.

In a step 22, the full machine learning model MLM is trained with second input data IP2 comprising examination images that are also extracted from said longitudinal dataset TDS. It is therein assumed that the model MLM comprises the first neural network NN1 and one or more further components that are placed behind the first neural network NN1 and configured to receive the first vector of learnt features z(t) as input. In one or more examples, the training 22 is launched once the pre-training is complete, but this is not compulsory.

The training 22 aims at configuring the model MLM to predict an information of pathology progression, in this example a severity grade from a single input image associated with a current time t at a future time t+$\Delta$t.

The second input data IP2 used for training the full model MLM are thus different from the first input data IP1. In particular, instead of a pair of consecutive examination images of a pair of images taken from a time series of the training data set, they only comprise the first image x(ti)of a pair, and an intermediate time $t_{mix}$, selected between the first time ti of the first image x(ti) et the second time ti+1 of the second image x(ti+1).

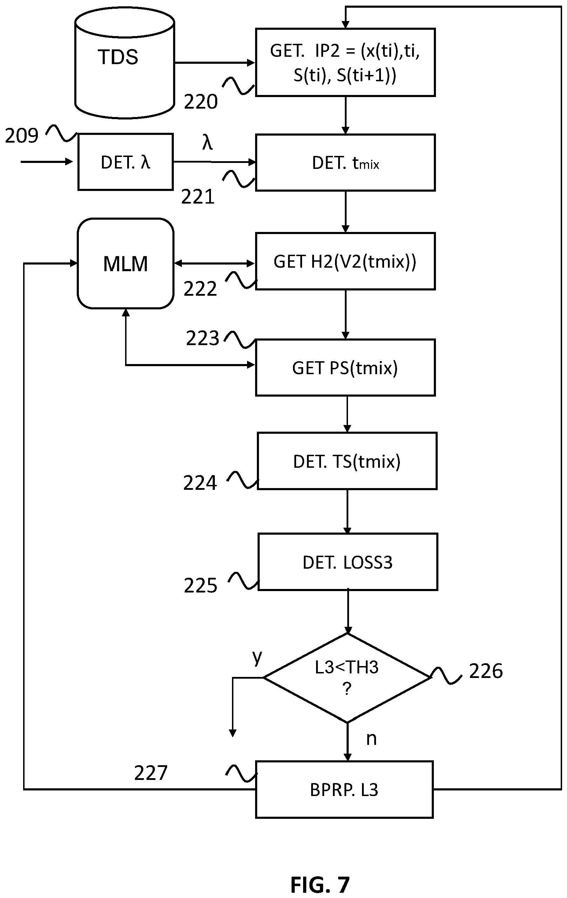

During training 22, for each new input image IP2 of a pair and chosen intermediate time tmix, a severity grade $PS(t_{mix})$ is predicted and a loss function l' is used to determine an error between the predicted severity grade $PS(t_{mix})$ and a target severity grade $TS(t_{mix})$ derived from the given profile of pathology progression I(t) which is applied to the first and second times ti, ti+1 associated respectively to the consecutive images of the input pair. The error is further backpropagated into the machine learning model MLM whose internal features or weights are updated accordingly. Training is repeated with other first images of input pairs IP2 from the training dataset TDS until a stopping criterion is reached, for example while the error is greater than a second predetermined threshold. An exemplary embodiment of this training step will be detailed hereinafter in relation with FIGs. 6 and 7.

In relation with FIGs. 3, 4A and 4B, the pre-training 21 is now detailed. In the example of FIG. 3, the first neural network NN1 comprises a backbone g1:n with n integer greater than 2, representing a number of layers of said backbone, where g1:k denotes the part of the neural network mapping the input data IP1 to an hidden representation at layer k. For example, NN1 is a fully connected layered network FCN, comprising n layers, with n an integer greater than 2, and for example equal to 7. It is configured to take a pair of consecutive examination images as input and to output a vector $z_1(t)$ of D features, with D an integer greater than 2. For example, when the input examination images are of size 256x 256 with three color channels RGB, the first neural network NN1 is configured to provide as output a first vector of features of size 64x4x4. As the size of information contained in the first vector of features is reduced with respect to the one of the input data, the first neural network is also referred to as an encoder.

More precisely, in one more examples, the first neural network ENC may comprise a stack of pre-activated residual blocks comprising each at least one convolutional layer, followed by a "relu" activation function, and a batch normalization function. In one or more examples, in each residual block, the residual feature map is calculated using a series of three $3\times3$ convolutions, the first of which always halves the number of the feature maps employed at the current residual block. In one or more examples, among the seven layers; the first six layers are composed of two residual blocks each and the latter deals with only one residual block. In one or more embodiments, the last three layers of the first neural network NN1 are eligible layers for temporal mix-up within the first neural network as will be described therein in relation with FIG. 4B.

In relation with FIG. 4A, pre-training 21 comprises the following sub-steps:

In 210, an input pair IP1 of consecutive examination images (x(ti), x(ti+1) of a patient is extracted from the training dataset TDS. It comprises a first image x(ti), a second image x(ti+1). In 211, an intermediate time $t_{mix}$ is determined within the time interval [ti,ti+1]. In one or more examples, $t_{mix}$ is determined by applying a linear interpolation between ti and ti+1 using a weighting coefficient $\lambda$, a real comprised within [0,1], using a mixing operator defined by $Mix_{\lambda}(a, b) = \lambda. a + (1 - \lambda). b$ with $\lambda \in$ [0, 1].

We thus have:

$$t_{mix} = Mix_{\lambda}(t_i,\ t_{i+1}) = \lambda. t_i + (1 - \lambda). t_{i+1} \qquad (3)$$

In one or more examples, $\lambda$ is randomly determined in sub-step 209 based on a given distribution law DL, for instance the Beta distribution law illustrated by FIG. 5A. The Beta distribution law defines several (7) distribution curves depending on a parameter $\beta$, which is a function of $\alpha$. In the following, we consider more particularly one of the distribution curves corresponding to $\beta = \alpha$ and $\lambda \sim$ Beta $(\alpha, \alpha)$, with $\alpha$ comprised within the interval [2,10].. They are bell-shaped, like a more or less flattened Gaussian, centered on the middle of the interval. Preferably, $\beta = \alpha$ is set equal to 2.

Use of such a distribution law allows to get a distribution of interpolation weighting factor $\lambda$ in the time interval and to ensure that various values will be used at successive iterations of the pre-training process. In one or more examples, a same value of $\beta$ is used for the whole pre-training of first neural network NN1.

In an optional sub-step 212, the first and second images of the input pair IP1 are mixed up, based on the weighting factor $\lambda$, to get an intermediate image x(tmix) associated with intermediate time $t_{mix}$.

The intermediate image $x(t_{mix})$ is obtained as follows:

$$x(t_{mix}) = Mix_\lambda(x(t_i),\ x(t_{i+1})) = \lambda.\,x(t_i) + (1-\lambda).\,x(t_{i+1}) \qquad (4)$$

This mix-up is inspired from a known technique introduced in Thulasidasan et al., in a document entitled "On mixup training: Improved calibration and predictive uncertainty for deep neural networks", published in journal "Neural Information Processing Systems", in 2019, as a simple regularization method to minimize overfitting in deep neural networks. This technique consists in linearly interpolating a mini-batch of random examples and their labels to transform the training set. Here, not only the images and labels, but also the associated times are also mixed up to create intermediates times and associated intermediate images.

In 213, the intermediate time $t_{mix}$ and the intermediate image $x(t_{mix})$ are presented to the first neural network NN1 and a first vector of D features $z_1(t_{mix})$ is obtained as output of the g1:n backbone of layers. It may be expressed as follows:

$$z_1(t_{mix)} = g1{:}n(Mix_\lambda(x(t_i),\ x(t_{i+1})). \qquad (5)$$

In 214, a target severity grade TS($t_{mix}$) at intermediate time $t_{mix}$ is determined based on the input severity grades $S(t_i)$ and $S(t_{i+1})$ associated with the first and second images of the input pair and on a given pathology progression profile I(t). In one or more examples, I(t) may be a linear ($I_{lin}$) or exponential interpolation function ($I_{exp}$) as illustrated by FIG. 5B.

Temporal mix-up of the input data allows to generate soft labels that modulate the learned decision boundaries, providing the model with more information. It also achieves data augmentation of the longitudinal dataset TDS as more input data may be obtained from a same number of series of follow up examination images of patients stored in the training data set. Finally, it may also contribute to better train the whole model by avoiding overfitting.

In 215, a value of severity grade PS($t_{mix}$) at intermediate time $t_{mix}$ is predicted based on the output first vector of features $z_1(t_{mix})$. In the example of FIG. 4A, this prediction is performed a first classification head H1, that is placed behind the first neural network NN1. In one or more examples, H2 comprises a feed forward multi-layered neural network, for instance of MLP type. For example, the first classification head H1 comprises two layers. A first layer L1 takes the D features of vector $z_1(t_{mix})$ as input and maps them to M<D internal features of a second layer L2, that maps them into one predicted value of severity grade PS($t_{mix}$), that is the output of the first classification head H1, H1 ($z_1(t_{mix})$)).

In 216, an error or loss $L_1$ is determined using a loss function $l$ between the predicted severity grade PS($t_{mix}$) and a target severity grade TS($t_{mix}$), as follows:

$$L_1 = l(I(t_{mix}), H_1(z(t_{mix})) \qquad (6)$$

wherein TS($t_{mix}$) = I($t_{mix}$) and PS($t_{mix}$) = $H_1(z_1(t_{mix}))$.

In one or more examples, a Binary Cross Entropy (BCE) loss function is used. Originally, BCE function are designed to work with binary input values and thus to solve binary issues (with two classes only). However, it may be adapted to several class issues by encoding the input values, in such a way to represent each of them as a sequence of binary values. In the example of FIG. 4A, the real values of predicted and target severity PS($t_{mix}$) and TS($t_{mix}$) are "hot encoded" in 217, that is the numerical value of each severity is encoded into a binary vector of size equal to the number of possible severity grades, namely 5, and their encoded values are used in 217 to determine the error $E_1$ using the BCE loss function.

In one or more examples, the first classification head $H_1$ comprises a last layer dedicated to perform an activation function (for instance sigmoid) needed for the BCE loss function. In an alternative, a BCE function with the activation function integrated (BCE with LOGIT) may be implemented instead of an activation layer in the classification head H1.

In 218, the error $L_1$ is compared with a given threshold TH1. If the error $L_1$ is greater or equal than the threshold TH1, the error L1 is backpropagated into the first neural network NN1 along with the first classifier H1, which will cause update of their internal weights or parameters, and a new iteration of training is triggered. Thus, sub-steps 210-219 are repeated while the error L1 is greater or equal than the threshold TH1.

In one or more examples, the machine learning architecture used for training the first neural network NN1 further comprises a regression head $R_1$. This is another neural network which is also simultaneously trained with the first neural network NN1 and the first classification head H1 to predict a time $Pt_{mix}$ by regression of the output first vector of features $z_1$ ($t_{mix}$). In one or more examples, this regression head R1 comprises, like H1, a feed forward artificial neural network, of Multi-Layer Perceptron (MLP) type, comprising two of more layers. A second error or loss $L_{t_{mix}}$ is evaluated in 216 between this predicted time $Pt_{mix}$ and a target intermediate time corresponding to the intermediate time $t_{mix}$ determined at sub-step 211. The second error $L_{t_{mix}}$ is, for example determined as follows:

$$L_{t_{mix}} = \|t_{mix} - \widetilde{t_{mix}}\|_2^2 \qquad (7)$$

wherein $\widetilde{t_{mix}}$ is the intermediate time predicted by the regression head R1 $t_{mix}=R_1(z_1(t_{mix}))$, from the first vector of features $z_1(t_{mix})$ output by the first neural network NN1 for the given input pair IP1, and

wherein $\|\ \|_2^2$ stands for Euclidean norm.

This time consistency error $L_{t_{mix}}$ is used to to evaluate the ability of the model to produce consistent predictions for the time signal across different image pairs.

The second error $L_{t_{mix}}$ is compared with a second threshold TH2 in 218'. In 219', if the second error $L_{t_{mix}}$ is greater or equal than the second threshold TH2, the error $L_{t_{mix}}$ is backpropagated to the first neural network NN1 along with the regression head R1 and the classification head H1 and a new iteration of pre-training is launched. Thus, while at least one of both errors $L_1$, $L_{t_{mix}}$ does not pass the test 218, 218', the pre-training is repeated with at least one new input pair IP1. This second error $L_{t_{mix}}$ allows to check that the first vector of features $z_1(t_{mix})$ generates a latent representation that really corresponds to the determined $t_{mix}$. Thus, it allows to ensure time consistency of the task for which the first neural network NN1 is pre-trained.

In another example, illustrated by FIG. 4B, optional sub-step 212 of determining the intermediate image $x(t_{mix})$ is not performed. As all the other sub-steps 209-211 and 213-219 are the same as in FIG. 4A, there are not described again. Instead of sub-step 212, an alternative sub-step 212' is executed, which comprises selecting one layer $I_k$ of the first neural network NN1 among a given subset of layers S, for example the three last layers $I_{N-3}$ to $I_N$, and applying the temporal mix-up to the internal features of this layer $I_k$ within the first neural network NN1.

This alternative mix-up is inspired from a known technique referred to as Manifold Mix-up, which is an extension of Mix-up to hidden representations, that was described in Verma et al., in a document entitled "Manifold mixup: Better representations by interpolating hidden states", published in International Conference on Machine Learning, in 2018. During training, a random layer k from a set S of eligible layers in a neural network is selected. It is assumed that this selected layer is configured to process two random data mini-batches (x, y) and (x', y'), until reaching layer k+1. The mix-up is then performed on these two intermediate mini-batches (gk (x), y) and (gk (x') , y'), continuing the forward pass with the mixed representation until the ending layer n of the neural network.

In the example of FIG. 4B, a layer $_kl$ is randomly selected among the three n-3 to n layers of the first neural network NN1, based on a uniform distribution. It is assumed that the layer $I_k$ comprises a first batch of features g1 :k(x(ti)) related to the first examination image x(ti) and the second batch of features g1:k(x(ti+1)) are related to the second examination image x(ti+1). Then, these features of layer $I_k$ are temporally mixed up k+1 as follows:

$$\text{Mix}_\lambda(g1{:}k(x(t_i)), g1{:}k(x(t_{i+1}))) = \lambda.g1{:}k(x(ti)) + (1-\lambda).gk{:}n(x(ti+1)). \qquad (8)$$

The mixed up features of layer Ik are then transferred to next layer $I_{k+1}$ and processed as follows :

$$gk{+}1{:}n(\text{Mix}_\lambda(g1{:}k(x(t_i)), g1{:}k(x(t_{i+1})))) \qquad . \qquad (9)$$

We have just described exemplary embodiments to pre-train the first neural network NN1 to perform a pretext task, or longitudinal pretext task, that encodes the pathology progression within time intervals between two consecutive examination images of a patient.

It should be noted that, although a complete iteration (sub-steps 210-219 or 210-219') of the pre-training process was described for an input pair of consecutive examination images, in practice, a batch comprising a plurality of input pairs, for example 60 input pairs, may be applied to the first neural network NN1 (or combination of neural networks NN1 + H1 + R1) before determining an error and backpropagating this error. This allows to save memory and computation time.

In one or more examples, a total error or loss L is then evaluated as follows:

$$L=E_{(x(t_i),x(t_{i+1}))\sim v}E_{\lambda\sim Beta(\alpha,\alpha)}E_{k\sim S}\big(l(H_1(z(t_{mix}),I(t_{mix})))+L_{t_{mix}} \qquad (10)$$

wherein:

- $E_{(x(ti),x(ti+1))\sim v}$ denotes an expectation, for example a mean, over the current batch or the entire training dataset $v$ of input pairs of consecutive examination images of patients included into the training dataset TDS,
- $E_{\lambda\sim Beta\alpha(\alpha,\alpha)}$ represents expectation over the mixing parameter $\lambda$, which is drawn from a Beta distribution with parameters, $\beta = \alpha$. As described above, the Beta distribution is a conjugate prior for the binary cross-entropy loss function, and it is used to model the uncertainty in the mixing parameter,,
- $E_{k\sim s}$ represents the expectation over the random subset of layers $l_k$ that are selected for an input image pair, when sub-step 212' is performed (Manifold temporal mix-up). The subset of eligible layers S of the first neural network NN1 is drawn from a probability distribution. It should be noted that in case mix-up is used in place of manifold mix-up, S is set to {0} in (7),
- $L1 = l(H_1(z(t_{mix}),I(t_{mix}))$ represents the first error or loss $L_1$ made by the neural network NN1, when processing the input mixed image pair $(x(t_i),x(t_{i+1}))$. The loss function used here is the binary cross-entropy loss function, which measures the difference between the predicted output $PS(t_{mix}) = H_1(z_1(t_{mix}))$ and the ground truth output $TS(t_{mix}) = I(t_{mix})$.

wherein $L_{t_{mix}}$ is the "time consistency" loss term or error, as defined above, which is added to the main error or loss $L_1$ in the training of the first neural network NN1.

In relation with FIGs. 6 and 7, the training 22 of the full machine learning model MLM is now detailed. In the example of FIG. 6, the machine learning model MLM comprises the pre-trained first neural network NN1 and at least a second neural network NN2. Both are placed in series, in such a way that output from the first neural network NN1, namely the first vector of learnt features $z_1(t)$, output by the first neural network NN1 is presented as input to the second neural network NN2. In the example of FIG. 6, the second neural network is configured to output a second vector of features $z_2(t+\Delta t)$, having the same size, or number D of features than the first vector $z_1(t)$. As the expected output of the full machine learning model MLM is a predicted severity grade PS(t) corresponding to an input future time t $\Delta t$, the model MLM further comprises a classification head H2, placed in series with the second neural network, that takes the second vector of features $z_2(t +\Delta t)$ as input and outputs the predicted severity grade PS(t+$\Delta t$).

In one or more examples, the second neural network NN2 is a time-aware machine learning model, for example a neural network configured to achieve a Neural Ordinary Differential Equations (NODE) approximation task by parametrizing the continuous dynamics of hidden units z $\in$ $R^n$ over time with t $\in$ R.

As known in the art, NODEs are able to model the instantaneous rate of change of $z_2$ with respect to t using a neural network u with parameters $\theta$:

$$\lim_{h\to 0}\frac{z(t+h)-z(t)}{h} = \frac{dz}{dh} = u(t,z,\theta) \qquad (11)$$

The analytical solution of (11) is given by:

$$z(t_1) = z(t_0) + \int_{t_0}^{t_1} u(t,z,\theta) = ODESolve(z(t_0),u,t_0,t_1,\theta) \qquad (12)$$

where [t0, t1] represents the time horizon for solving the ODE, u a neural network, and $\theta$ stands for trainable parameter of u.

By using a NODE as a black-box as disclosed by Chen et al.. in the document entitled "Neural Ordinary Differential Equations", published in 2018, at URL By using a NODE as a black-box as disclosed by Chen et al. in the document entitled "Neural Ordinary Differential Equations", published in 2018, at URL https://doi.org/10.48550/AR XIV.1806.07366 or //arxiv.org/abs/1806.07366, an Initial Value Problem (IVP) can be solved and the hidden state, i. e. the output vector of features $z_2(t)$; calculated at any desired time t using Eq. (12).

, an Initial Value Problem (IVP) can be solved and the hidden state, i. e. the output vector of features $z_2(t)$; calculated at any desired time t using Eq. (12).

We can differentiate the solutions of the ODE solver with respect to the parameters $\theta$, the initial state $z(t_0)$ at initial time $t_0$, and the solution at time t. For example, this can be achieved by using an adjoint sensitivity method disclosed by Chen.

When applied to the present case, through the latent representation or vector of features $z_2(t)$ of a given input examination image x(ti), an IVP that aims to solve the ODE from first time ti to second time ti+1, is defined as follows:

$$z_2\dot{(t)}=u(z_2(t),t,\theta) \qquad (13)$$

With the initial value $z_2(t_i) = z_1(t_i)$, $z_1$(ti) being the output of the longitudinal pretext task carried out by the first neural network NN1.

In relation with FIG. 7, training 22 comprises the following sub-steps:

In 220, input data IP2 are extracted from the training dataset TDS. They comprise at least one first image x(ti) of a pair of consecutive examination images of a region of a same patient, the first time ti associated with the first image x(ti) and the severity grades S(ti), S(ti+1) associated with both images of the pair.

In 221, an intermediate time $t_{mix}$ is determined within the time interval [ti,ti+1]. In one or more examples, $t_{mix}$ is determined by applying a linear interpolation between ti and ti+1 using a weighting coefficient $\lambda$, wherein $\lambda$ is randomly determined as described above in relation with FIGs 4A and 4B (sub-step 209) based on a given distribution law DL, for instance the beta distribution law illustrated by FIG. 5. It should be noted that the same values of $\beta$ and $\alpha$ are chosen as for pre-training 21. Preferably, $\beta = \alpha = 2$.

In 222, the first image x(ti) is presented to the machine learning model MLM, that it is applied as input to the neural network NN1. NN1 outputs a first vector of features $z_1$(ti) which is applied as input to the second neural network NN2. In parallel, the determined intermediate time $t_{mix}$ is applied as a parameter to NN2. NN2 outputs a second vector $z_2(t_{mix})$. In one or more examples, NN2 is configured to solve differential equation (3) presented above.

It should be noted that instead of solving the ODE from first time ti to second time ti+1 using Eq.3, the ODE is solved for intermediate time $t_{mix}$ as follows:

$$z_2\dot{(t)}=u(z_2(t),t_{mix},\theta) \quad (14)$$

with the initial value $z_2(t_i) = z_1(t_i)$

This approach could be applied to any other time-aware model than the NN2 (NODE) used in the example of FIG. 6. For instance, a Time-LSTM model, described in Baytas et al, entitled "Patient subtyping via time-aware lstm networks", published in 2017, at the URL https://doi.org/10.11.15/3097983.3097997Time-LSTM is a variant of the Long Short-Term Memory neural network (LSTM), in which time gates were incorporated. This enables the LSTM to learn to forget or remember information as a function of the time intervals between input sequences.

The output second vector $z_2(t_{mix})$ is then applied in 223 to the classifier head $H_2$ that is configured to predict a severity grade $PS(t_{mix})$ based on the input second vector of features $z_2$(tmix).

$t_{mix}$ is further used in 224 to evaluate a target severity grade $TS(t_{mix})$ which will be as supervision or label for training the full model MLM.

In one or more examples, the second classifier head $H_2$ comprises, like H1, a feed forward multi layered neural network, for instance of MLP type. For example, the second classification head H2 comprises two layers. A first layer L1 takes the D features of vector $z_2(t_{mix})$ as input and maps them to M<D internal features of a second layer L2, that maps them into one predicted value of severity grade $PS(t_{mix})$, that is the output of the second classification head H2, $H_2(z_2(t_{mix}))$.

It should be noted that the second classifier head H2, unlike H1, is part of the machine learning model MLM.

In 224, a target severity grade $TS(t_{mix})$ at the intermediate time $t_{mix}$ is determined, as already described above in relation with FIGs 4A and 4B, from the pathology progression profile I(t) and the severity grades respectively associated with the first and second images of the pair, the input image x(ti) is originated from. Preferably, the same pathology progression profile I(t) is used in the training step 22 as in the pre-training step 21.

In 225, an error or loss $L_3 = l'(TS(t_{mix}), PS(t_{mix}))$ is determined using a loss function, between the predicted severity grade $PS(t_{mix})$ and a target severity grade $TS(t_{mix})$. In one or more examples, a Binary Cross Entropy (BCE) loss function is used, as already described above for the first classification head $H_1$. In this case the integer values of predicted and target severity grades $PS(t_{mix})$ and $TS(t_{mix})$, that range between 0 and 4) are beforehand "hot encoded" (not represented), that is the numerical value of each severity is encoded into a binary vector of size equal to the number of possible severity grades, namely 5, and their encoded values are used in 225 to determine the error L3 using the BCE loss function.

In 226, the error L3 is compared with a given threshold TH3. If the error L3 is greater or equal than the threshold TH3, the error I' is backpropagated in 227 into the machine learning model MLM, which will cause update of its internal weights or parameters, namely those of NN1, NN2 and $H_2$, and a new iteration of training is triggered. Thus, sub-steps 220-2227 are repeated while the error I' is greater or equal than the threshold TH3.

It should be noted that, in the same way as for pre-training 21, an iteration may be performed for a batch comprising a plurality of input examination images IP2 and the error L3 may only be determined once all the examination images of the batch have been applied to the model MLM.

Once training phase 22 is over, the trained machine learning model MLM is then charged into a ML agent which can then be used in an operation phase. Of course, the model MLM can be re-trained from time to time if necessary, in order to improve performance.

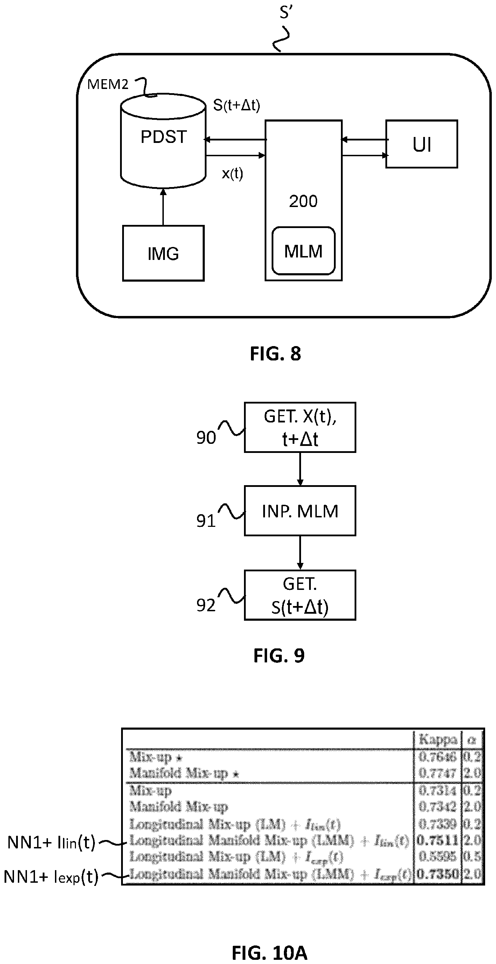

In relation with FIG. 8, an apparatus 200 for using the machine learning model MLM to predict an information representative of a progression of a pathology is presented, in an exemplary context in which it may be deployed.

In the example of FIG. 8, said apparatus 200 is integrated into a system S for processing examination images of patients. The system S comprises imaging means IMG configured to capture examination images of patients according to at least one imaging modality. The examination images are stored in a memory MEM2, in a patient dataset PDST that may be organized as a database. In one or more examples, the system S further comprises a user interface UI, for example displays means such as a monitor, through which the examination images captured by the imaging means IMG and/or prediction results output by the apparatus 200 may be displayed to a user, such as a practitioner. Apparatus 200 further comprises communication means to communicate with other components of the system S, comprising at least the memory MEM2 and the user interface UI. The system S may be installed in a clinical environment, such as a hospital.

Apparatus 200 is configured for predicting a severity grade representative of a pathology progression at a future time (t+$\Delta$t) from one or more examination images of an area of a patient captured at a current time (t). In one or more examples, the trained model MLM is charged into a memory (not represented) of apparatus 200, which further comprises means for presenting the one or more examination images and said future time as input to the trained model MLM, and for obtaining a predicted severity grade for said future time as output.

In one or more examples, the apparatus 200 is configured to implement a method for predicting a severity grade representative of a pathology progression at a future time (t+$\Delta$t) from one or more examination images of an area of a patient captured at a current time (t), that will now be presented in relation with FIG. 9.

In a step 90, one or more examination images of a region of a patient, for example a CFP image of part of an eye of the patient, associated with a time t, are obtained, for example from the imaging means IMG of the system S or from the patient dataset PDS or from another source. A future time t+$\Delta$t, for instance equal to t+ 6months, t + 1 year, t+ 2 years etc, is obtained, for example, selected by a practitioner through the user interface UI.

In a step 91, the examination image(s) $x_i(t)$ and the future time t+$\Delta$t are presented as input to the trained model MLM.

In a step 92, a predicted severity grade $S_i(t+\Delta t)$ is obtained for said future time as output from the trained MLM.

It should be noted that any future time t+$\Delta$t within a time interval (ti, ti+1) used for training the machine learning model, may be input.

This prediction of pathology progression may help the practitioner handle the patient case. For example, the predicted severity grade $S_i(t+\Delta t)$ may be used by the for scheduling a next visit of the patient.

In relation with FIGs 10A- 10C, examples of experimentations and associated results are now presented.

Database

In an example, the apparatus 100 trained and evaluated the machine learning model MLM on public training database OPHDIAT. This is a large CFP database collected from the Ophthalmology Diabetes Telemedicine network consisting of examinations acquired from 101,383 patients between 2004 and 2017. Out of 763,848 interpreted CFP images, 673,017 were assigned a DR severity grade, and the others were non-gradable. Patients range in age from 9 to 91, and image sizes vary from 1440 $\times$ 960 to 3504 $\times$ 2336 pixels. Each examination includes at least two eye images. To limit consecutive pairs without progression, 10412 patients were selected with at least one severity change. Each patient had 2-5 scans, averaging 3.43, spanning an average interval of 4.86 years.

This dataset was further divided into training (60%), validation (20%), and test (20%) based on patients. One image per eye was randomly selected for each examination, resulting in 49578 pairs.

In a first experiment, the pre-trained first neural network NN1 was used alone, without the second neural network NN2, to predict whether an eye without DR at an initial visit would later be graded as having Mild+ DR within two years.

8,111 patients and 13,936 eyes fitting this criterion in the training dataset were used for the training. For the DR assignment, a specific test set consisted of patients assigned the same grade from two ophthalmologists, resulting in 9,734 eyes of 4,996 patients. All the timestamps initially expressed in days were normalized by 2$\times$365.

Implementation details

Regarding architecture of the first neural network NN1, a stack of 2 pre-activated residual blocks (with ReLu and Batch normalization) was employed. In each residual block, the residual feature map was calculated using a series of three 3$\times$3 convolutions, the first of which always halves the number of the feature maps employed at the present scale. The first neural network NN1 comprises 7 layers. The first six layers are composed of two residual blocks and the latter deals with only one residual block. This architecture allows to output a first vector of features providing a final latent representation of size $64 \times 4 \times 4$. The last three layers of the first neural network were used as eligible layers for performing Manifold Mix-up, as previously explained in relation with FIG. 4B.

More generally, the different neural networks forming the whole machine learning model MLM, were trained for 200 epochs by the AdamW optimizer, OneCycleLR as a scheduler, weight decay of10-4, and a batch size of input training data of 128 items, using an NVIDIA A6000 GPU with the PyTorch development framework. Concerning the NODE, the Pytorch package Torchdiffeq was used. This library provides ODE solvers, backpropagation through ODE solutions and a support of an adjoint method for solving an ordinary differential equation with a constant memory cost.

In an exemplary architecture, the second neural network NN2, NODE is a combination of dense layers followed by a tanh activation function and the adjoint method with "dopri5" as a solver. The adjoint method is for example the Dormand-Prince (RKDP) method or DOPRI method, which is an embedded method for solving ordinary differential equations . The loss in each experiment was used to monitor the machine learning model's performance on the validation set, the best one was kept.

Experiments for DR severity assessment by the first neural network NN1 (pretext task) using mixing training.

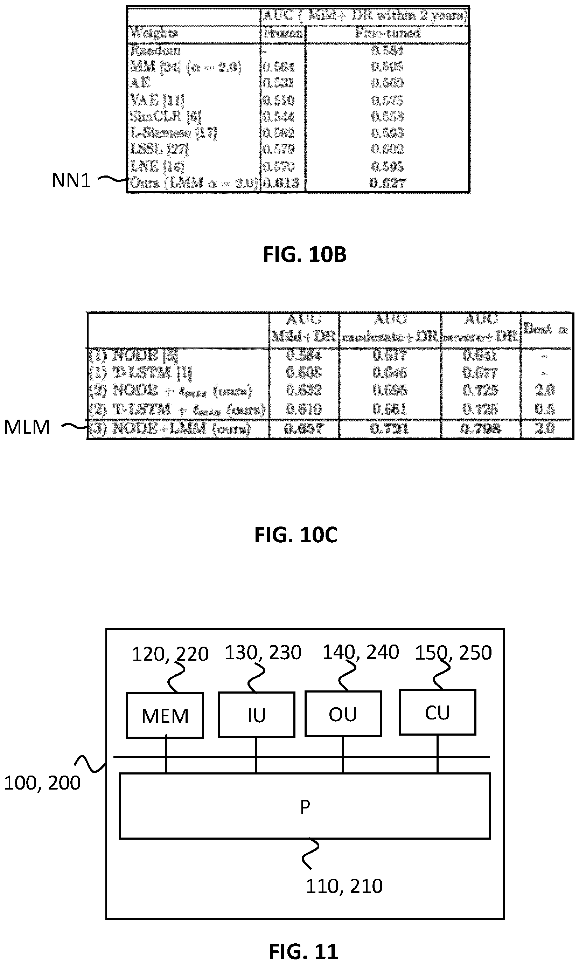

In relation with FIG. 10B, performances of the pre-trained first neural network NN1 described therein when longitudinal mix-up or longitudinal manifold mixup is used, are compared in a table with other scenarios where the same first neural network NN1 is applied classical mix-up or manifold mix-up.

The two first lines of the table refer to experiments made respectively for longitudinal mix-up and longitudinal manifold mix-up, based on training batches comprising input pairs of nonconsecutive images from the longitudinal dataset TDS. For the 6 next lines, training batches only comprise fixed pairs of consecutive images. In order to augment the number of examples presented to the first neural network NN1, permutations of input pairs were made between training batches which allowed to have pairs from distinct patients in a same batch.

Results reported in the table of FIG. 10A are based on a Quadratic-weighted Kappa which is a measure or coefficient, usually referred to as Cohen's Kappa Score, commonly used to assess the effectiveness of machine learning classification.

As shown by table, the two first experiments set apart, the pre-trained NN1 described therein when using longitudinal manifold mix-up with linear profile of pathology progression ($l_{lin}(t)$) outperforms all the other scenarios.

Experiments to evaluate the quality of feature extraction.

In relation with FIG. 11B, the performance of several other pre-trained models were compared with the pre-trained first neural network NN1, LMM described therein. The general or downstream task to be performed was to predict a severity grade at a fixed future time of ti +2 years, from input examination images associated with a severity grade of 0 at first time ti. The results are provided in the table of FIG. 10B from both linear evaluation and fine-tuning.

Linear evaluation is a known technique that involves training a linear classifier on representations or vectors of features learnt by a pre-trained neural network. The pre-trained neural network is frozen, i.e. its weight parameters are not updated while the linear classifier is being trained.

Linear evaluation is a simple and effective technique for assessing the quality of representations learned by a neural network. It is also useful for adapting a neural network to a new task without the need for a large set of labeled data.

Fine tuning is another technique that involves training a pre-trained neural network on a new set of labeled data. The pre-trained neural network is used as a starting point, and its internal parameters are updated during training to adapt to the new or general task.

Fine tuning is a stronger technique than linear evaluation, as it allows the neural network to learn the specifics of the new task as proposed.

Evaluation was made with the Area Under the receiver operating characteristic Curve (AUC). The linear evaluation was conducted by training a classification head (having typically one single layer) on top of the pre-trained and frozen encoder (i. e. the first neural network pre-trained to output a first vector of learnt features) and trained using the same set-up that was tried with the first neural network NN1 , LMM described therein. In the table of FIG. 10B, performances of NN1 are compared with those of classical feature extractors, namely Auto-Encoder (AE), Variational Auto-Encoder (VAE), Simple Framework for Contrastive Learning of Visual Representations (SIMCLR), and of known longitudinal pretext tasks, namely Longitudinal Siamese (LS), Longitudinal self-supervised learning (LSSL) and Self-supervised longitudinal neighborhood embedding (LNE).

As can be seen, the encoder NN1 described therein outperforms all the classical encoders and known longitudinal pretext tasks.

Experiments using temporal mix-up for time-aware models.

In relation with FIG. 10C, performances of the trained model MLM are compared with another time-aware model T-LSTM, known in the art, and introduced in a document by Baytas et al., already cited above , in order to demonstrate the effectiveness of using intermediate times tmix in the predictive model described therein.

Both time-aware models take as input the latent representation provided by the first vector of features $z_1$ti) output by the pre-trained neural network NN1 described therein and the time difference between xti and xti+1 ($\Delta$t) and they are requested to predict the latent representation of vector $z_2(t_i+1)$. For the NODE, it was performed by the mean of IVP defined in Eq.(14) while for T-LSTM], the LSTM gates were modulated by $\Delta$t to produce the future $z_2$(ti+1). Three training setups were tested:

1 - No pretext task NN1 is used. One input examination image x(ti) of a patient associated with a current time ti and the future time ti+1 of the next examination image image captured for this patient, are directly applied to the NN2 (combined with H2) described therein or other time-aware model. Loss L3 = l'($H_2(z_2$t), S(ti+1))) is evaluated to train the models.

2. No pretext task NN1 is used either. Similarly to first set-up, we applied a single image x(t), but instead of giving the time of the next examination, an intermediate $t_{mix}$ between ti and ti+1 was presented to the models and trained with loss L3 = l'($H_2(z_2(t_{mix})$,, I($t_{mix}$)).

3. The same scenario is used as in the second set-up, but applied to the whole machine learning model, either the model MLM described therein or with another time-aware neural network in place of NN2.

As shown by the table of FIG. 10C, the best results are obtained with the model MLM described therein, which proves is effective ability to capture disease progression. Concerning the use of longitudinal mix-up, results indicate that it is beneficial for both time-aware models. This confirms that use of an intermediate time $t_{mix}$ both provides data augmentation in the context of disease progression for Time-Aware models and contributes to regularize training of the time aware model. The fact that the set-up of the model MLM described therein performs better than other configurations supports the idea that combined use of a longitudinal pretext task and temporal mixup is beneficial to solve time-related problems.

A person of skill in the art would readily recognize that steps of various above-described methods can be performed by programmed computers. Each step may be implemented by a different software module or as a part of a common software module. Many embodiments are possible and accessible to the skilled person knowledgeable in software engineering.

Figure 11 illustrates an example of the hardware structure of an apparatus 100 for training a machine learning model to predict an information representative of a pathology progression as described therein and/or of an apparatus 200 for predicting an information representative of a pathology progression a configuration by using a trained machine learning model as described therein. In this example, the apparatus 100, 200 is configured to implement all the steps of the aforementioned method as described herein. Alternatively, it could also implement only some of these steps.

Related to FIG. 11, the apparatus 100, 200 comprises at least one processor 110, 210 and at least one memory 120, 220. The apparatus 100, 200 may also comprise one or more communication interfaces. In this example, the apparatus 100, 200 comprises network interfaces 130, 230 (e.g., network interfaces for wired/wireless network access, including Ethernet interface, WIFI interface, etc.) connected to the processor 110, 210 and configured to communicate via one or more wired/unwired communication links and user interfaces 140, 240 (e.g., keyboard, mouse, display screen, etc.) connected to the processor. Apparatus 100, 200 may also include one or more RDR media drives 150 for reading a computer-readable storage medium (e.g. a digital storage disc (CD-ROM, DVD, Blue Ray, etc.), a USB stick, etc.). Processor 110, 210 is connected to each of the other aforementioned components in order to control their operation.

Memory 120, 220 may comprise random access memory (RAM), cache memory, nonvolatile memory, backup memory (e.g., programmable or flash memories), read-only memory (ROM), hard disk drive (HDD), solid-state drive (SSD) or any combination thereof. The ROM of memory 120, 220 can be configured to store, among other things, an operating system of device 100 and/or one or more computer program codes of one or more software applications. The RAM of memory 120 can be used by processor 110, 220 for temporary data storage.

The processor 110, 210 can be configured to store, read, load, execute and/or otherwise process instructions stored in a computer-readable information storage medium and/or in memory 120, 220 so that, when the instructions are executed by the processor, the device 100 executes one or more or all of the steps of the method described herein. Means implementing a function or set of functions may correspond in this document to a software component, a hardware component or a combination of hardware and/or software components, capable of implementing the function or set of functions, as described below for the means concerned.

An embodiment relates to an information carrier readable by a data processor, comprising instructions of a program as mentioned above. The information carrier may be any hardware means, entity or apparatus, capable of storing instructions of a program as mentioned above. Usable program storage media include ROM or RAM memory, magnetic storage media such as magnetic disks and magnetic tapes, hard disks or optically readable digital data storage media, or any combination thereof.

In some cases, the computer-readable storage medium is non-transitory. In other cases, the information carrier may be a transient medium (e.g., a carrier wave) for transmitting a signal (electromagnetic, electrical, radio or optical signal) carrying program instructions. This signal can be routed via a suitable wired or wireless transmission medium: electric or optical cable, radio or infrared link, or by other means.

An embodiment also relates to a computer program product comprising a computer-readable storage medium on which program instructions are stored, the program instructions being configured to cause the apparatus 100, 200 to implement some or all of the steps of one or more of the methods described herein when the program instructions are executed by one or more processors and/or one or more programmable hardware components of the apparatus 100, 200.

According to one or more embodiments, apparatus 200 is itself integrated into a host device, for example the system S for processing examination images of a patient as described herein. This system, too, may be implemented by one or more physically separate machines and have the overall architecture of a computer and the hardware structure of a computer as previously described for apparatus 200 in connection with FIG. 12. In one or more examples, apparatus 100 is not integrated into system S, but into a distant server. Once trained, the machine learning model MML is charged into apparatus 200 of system S, that may be installed in a clinical environment. In an alternative, apparatus 100 is integrated into system S, that is training, or at least re-retraining of the machine learning model MLM may be performed in situ.

The above-mentioned embodiments and their variants each have numerous advantages. They make it possible to harness the power of machine learning in a method and apparatus for predicting progression of a pathology over time, using a machine learning model, that has been trained with a longitudinal training dataset comprising series of consecutive examination images of patients. The trained machine learning model described therein is time aware and able to output a value of an information of pathology progression at a future time from a single examination image of the patient at a current time presented as input.

The methods, apparatus and system presented therein find many applications, in particular, for assisting practitioners in handling a patient's case, when assessing how quickly the disease progresses, scheduling a next visit, deciding, deciding on best suited treatment protocol, etc.

## Claims

1. Method for training a machine learning model (MLM) for predicting information representative of a pathology progression (PS(t)) at a future time ($t+\Delta t$) from one or more examination images (x(t)) of a region of interest of a patient captured at a current time (t), said method comprising:

   - Getting (E0) a training dataset (TDS), comprising time series of consecutive examination images of said region of interest from patients associated with labels, said labels (S(t)) comprising said information representative of a pathology progression,
   - Training (E1, 21, 22) the machine learning model (MLM) with inputs from said training dataset comprising, at least one of said inputs comprising a pair of consecutive examination images comprising a first image ($x(t_i)$) associated with a first time ($t_i$) and first label ($S(t_i)$), a second image ($x(t_{i+1})$) associated with a second time ($t_{i+1}$) and a second label ($S(t_{i+1})$), to output a target intermediate label ($TS(t_{mix})$) at an intermediate time selected within a time interval ($[t_i, t_{i+1}]$) between the first and second examination images, said target intermediate label ($TS(t_{mix})$) being determined for said intermediate time ($t_{mix}$), based on a given pathology progression profile (I(t))

2. Method according to the preceding claim, wherein, said machine learning model (MLM) comprising a first neural network (NN1) and a second neural network (NN2), said training (E1) comprises pre-training (21) at least the first neural network (NN1) of the model with said input data from said training dataset (TDS) to output a first vector ($z_1(t)$ of features representative of the pathology progression on a time interval between the first time associated with a first image of the input pair and the intermediate time ($t_{mix}$), and
   wherein said training (E1) further comprises training (22) the machine learning model (MLM) by using said first vector of features ($z_1(t)$) as input to the second neural network (NN1).

3. Method according to the preceding claims, wherein said pre-training (21) comprises selecting said intermediate time ($t_{mix}$) (211) by mixing-up the first and second times of the consecutive examination images of the input pair, by linear interpolation, according to a given distribution function (DL).

4. Method according to claim 3, wherein the pre-training (21) further comprises determining (212) an intermediate image

$(x(t_{mix}))$ at the selected intermediate time $(t_{mix})$ by performing linear interpolation between the first and second images of the input pair.

5. Method according to claim 3, wherein the first neural network comprising a plurality of connected layers, said pre-training (21) further comprises mixing up (212') a first batch of internal features of at least one of said layers associated with the first image with a second batch of internal features of at least one of said layers associated with the second image.

6. Method according to any one of claims 2 to 5, wherein said pre-training (21) further comprises:

- obtaining (215°) a predicted information of pathology progression $(PS(t_{mix}))$ by presenting said first vector of features to a third neural network (H1), configured to be trained simultaneously with the first neural network (NN1) to output the predicted information of pathology progression $(PS(t_{mix}))$,
- determining at least one error $(L_1)$ between said predicted information of pathology progression $(PS(t_{mix}))$ and said target information of pathology progression $(TS(t_{mix}))$,
- backpropagating said error to the first neural network (NN1) while at least one stopping criterion based on at least said error $(L_1)$ has not been reached.

7. Method according to claim 6, wherein the pre-training (21) of the at least one first neural network comprises;

- determining (216'), based on a second loss function, a second error $(L_{tmix})$ between the intermediate time $(t_{mix})$ and a predicted intermediate time $(Pt_{mix})$ determined by regression from the output first vector of features $(z_1(t_{mix}))$, and
- backpropagating (219') the second error while at least one stopping criterion based on at least said second error has not been reached.

8. Method according to any one of claims 2 to 7, wherein the training (22) comprises:

- getting (220) second input data (IP2) from said training dataset (TDS), at least one of said input data comprising a first image (x(ti) of a pair of consecutive examination images (x(ti), x(ti+1)) from the training dataset, and a first associated label (S(ti)),
- selecting (221) an intermediate time $(t_{mix})$ between the first time and a second time of a second image of the pair, by linear interpolation, according to a given distribution function (DL), and
- presenting (222) said second input data to the first neural network (NN1) and getting a first vector of features $(z_1(ti))$ associated with said first time (ti) as output,
- presenting (222) said first vector of features and said intermediate time $(t_{mix})$ as input to the second neural network and obtaining a second vector of features $(z_2(t_{mix}))$ as output, said second vector of features having a same size as the first vector of features.
- determining (223) a target intermediate label $(TS(t_{mix}))$ associated with said intermediate time $(t_{mix})$ based on said given pathology progression profile (I(t)),
- predicting an output intermediate label $(PS(t_{mix}))$, said output intermediate label being obtained by regression from the second vector of features $(z_2(t_{mix}))$, and
- backpropagating a third error based at least on a third function loss between the output intermediate label and the target intermediate label, while a least one stopping criterion based at least on this third error has not been reached.

9. Method according to the preceding claim, wherein the second neural network comprises a Neural Ordinary Differential Equation, NODE, network, that is trained to output the second vector of features by solving a differential equation parametrized by the intermediate time.

10. Method for predicting an information representative of a pathology progression at a future time $(t+\Delta t)$ from one or more examination images of a region of interest of a patient captured at a current time (t), said method comprising:

- obtaining (90) the one or more examination images at said current time and selecting the future time,
- presenting (91) the one or more examination images and said future time as input to a trained machine learning model (MLM), said machine learning model (MLM) having been trained with inputs from a training dataset comprising at least one of said inputs comprising a pair of consecutive examination images comprising a first image (x(ti)) associated with a first time (ti) and first label (S(ti)), a second image (x(ti+1)) associated with a second time (ti+1) and a second label (S(ti+1)), to output a target intermediate label ($TS(t_{mix})$) at an intermediate time selected within a time interval ([ti,ti+1]) between the first and second examination images, said target intermediate label ($TS(t_{mix})$) being determined for said intermediate time ($t_{mix}$), based on a given pathology progression profile (I(t)), and
- obtaining (92) a predicted information ($PS(t+\Delta t)$ of pathology progression for said future time as output.

11. Apparatus (100) for training at least one AI module for predicting an information representative of a pathology progression at a future time ($t+\Delta t$) from one or more examination images (for instance one image 2D + a 3D volume) of an area of a patient captured at a current time (t), said apparatus comprising means for:

- getting a training dataset, comprising time series of (follow-up) examination images of said area from patients associated with labels, said labels comprising said information representative of a pathology progression,
- training the machine learning model (MLM) with inputs from said training dataset comprising, at least one of said inputs comprising a pair of consecutive examination images comprising a first image ($x(ti)$) associated with a first time (ti) and first label (S(ti)), a second image ($x(ti+1)$) associated with a second time (ti+1) and a second label (S(ti+1)), to output a target intermediate label ($TS(t_{mix})$) at an intermediate time selected within a time interval ([ti,ti+1]) between the first and second examination images, said target intermediate label ($TS(t_{mix})$) being determined for said intermediate time ($t_{mix}$), based on a given pathology progression profile (I(t))

12. Apparatus (100) according to the preceding claim, wherein the means comprises

- at least one processor; and
- at least one memory including computer program code, the at least one memory and computer program code configured to, with the at least one processor, cause the performance of the apparatus.

13. Apparatus (200) for predicting an information representative of a pathology progression at a future time ($t+\Delta t$) from one or more examination images (for instance one image 2D + a 3D volume) of a region of interest of a patient captured at a current time (t),
said apparatus comprising means for:

- obtaining the one or more examination images at current time and selecting the future time,
- presenting the one or more examination images and said future time as input to a trained machine learning model (MLM), said machine learning model (MLM) having been trained with inputs from a training dataset comprising at least one of said inputs comprising a pair of consecutive examination images comprising a first image ($x(ti)$) associated with a first time (ti) and first label (S(ti)), a second image ($x(ti+1)$) associated with a second time (ti+1) and a second label (S(ti+1)), to output a target intermediate label ($TS(t_{mix})$) at an intermediate time selected within a time interval ([ti,ti+1]) between the first and second examination images, said target intermediate label ($TS(t_{mix})$) being determined for said intermediate time ($t_{mix}$), based on a given pathology progression profile (I(t)), and
- obtaining a predicted information ($PS(t+\Delta t)$ of pathology progression for said future time as output.

14. Apparatus according to the preceding claim, wherein the means comprises

- at least one processor; and
- at least one memory including computer program code, the at least one memory and computer program code configured to, with the at least one processor, cause the performance of the apparatus.

15. A computer program comprising instructions which, when the program is executed by a computer, cause the computer to carry out the steps of the method of any of claims 1 to 10.

**FIG. 1**

**FIG. 2**

**FIG. 3**

EP 4 535 233 A1

**FIG. 4A**

**FIG. 4B**

**FIG. 5A**

**FIG. 5B**

**FIG. 6**

FIG. 7

S'

**FIG. 8**

**FIG. 9**

| | Kappa | $\alpha$ |
|---|---|---|
| Mix-up ⋆ | 0.7646 | 0.2 |
| Manifold Mix-up ⋆ | 0.7747 | 2.0 |
| Mix-up | 0.7314 | 0.2 |
| Manifold Mix-up | 0.7342 | 2.0 |
| Longitudinal Mix-up (LM) + $I_{lin}(t)$ | 0.7339 | 0.2 |
| Longitudinal Manifold Mix-up (LMM) + $I_{lin}(t)$ | **0.7511** | 2.0 |
| Longitudinal Mix-up (LM) + $I_{exp}(t)$ | 0.5595 | 0.5 |
| Longitudinal Manifold Mix-up (LMM) + $I_{exp}(t)$ | **0.7350** | 2.0 |

NN1+ $I_{lin}(t)$

NN1+ $I_{exp}(t)$

**FIG. 10A**

| Weights | AUC ( Mild + DR within 2 years) | |
| | Frozen | Fine-tuned |
| --- | --- | --- |
| Random | - | 0.584 |
| MM [24] ($\alpha = 2.0$) | 0.564 | 0.595 |
| AE | 0.531 | 0.569 |
| VAE [11] | 0.510 | 0.575 |
| SimCLR [6] | 0.544 | 0.558 |
| L-Siamese [17] | 0.562 | 0.593 |
| LSSL [27] | 0.579 | 0.602 |
| LNE [16] | 0.570 | 0.595 |
| Ours (LMM $\alpha = 2.0$) | **0.613** | **0.627** |

NN1

**FIG. 10B**

| | AUC Mild+DR | AUC moderate+DR | AUC severe+DR | Best $\alpha$ |
| --- | --- | --- | --- | --- |
| (1) NODE [5] | 0.584 | 0.617 | 0.641 | - |
| (1) T-LSTM [1] | 0.608 | 0.646 | 0.677 | - |
| (2) NODE + $t_{mix}$ (ours) | 0.632 | 0.695 | 0.725 | 2.0 |
| (2) T-LSTM + $t_{mix}$ (ours) | 0.610 | 0.661 | 0.725 | 0.5 |
| (3) NODE+LMM (ours) | **0.857** | **0.721** | **0.798** | 2.0 |

MLM

**FIG. 10C**

120, 220   130, 230   140, 240   150, 250

| MEM | IU | OU | CU |

100, 200

P

110, 210

**FIG. 11**

Europäisches
Patentamt

European
Patent Office

Office européen
des brevets

# EUROPEAN SEARCH REPORT

**Application Number**

EP 23 30 6730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ANEGONDI NEHA ET AL: "Deep Learning to Predict Geographic Atrophy Area and Growth Rate from Multimodal Imaging", OPHTHALMOLOGY RETINA 20171101 ELSEVIER INC USA, vol. 7, no. 3, 1 March 2023 (2023-03-01), pages 243-252, XP093144344, ISSN: 2468-6530, DOI: 10.1016/j.oret.2022.08.018 * pages 243-249 * | 1-15 | INV. G06N3/045 G16H50/20 |
| A | EMRE TAHA ET AL: "TINC: Temporally Informed Non-contrastive Learning for Disease Progression Modeling in Retinal OCT Volumes", 16 September 2022 (2022-09-16), 20220916, PAGE(S) 625 - 634, XP047633731, [retrieved on 2022-09-16] * pages 625-632 * | 1-15 | |
| A | WO 2020/226696 A1 (HUAWEI TECH CO LTD [CN]) 12 November 2020 (2020-11-12) * paragraphs [0001] - [0090] * | 1-15 | **TECHNICAL FIELDS SEARCHED (IPC)** G06N G16H |
| A | WO 2022/094629 A1 (GENENTECH INC [US]; HOFFMANN LA ROCHE [CH] ET AL.) 5 May 2022 (2022-05-05) * paragraphs [0002] - [0102] * | 1-15 | |

The present search report has been drawn up for all claims

| Place of search | Date of completion of the search | Examiner |
|---|---|---|
| The Hague | 28 March 2024 | Moran, Matthew |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 30 6730**

This annex lists the patent family members relating to the patent documents cited in  the above-mentioned European search report.
The members are as contained in the European Patent Office EDP file on
The European Patent Office is in no way liable for these particulars which are merely given for the purpose of information.

**28-03-2024**

| Patent document cited in search report | | Publication date | Patent family member(s) | | Publication date |
|---|---|---|---|---|---|
| WO 2020226696 | A1 | 12-11-2020 | CN | 114303177 A | 08-04-2022 |
| | | | EP | 4042318 A1 | 17-08-2022 |
| | | | WO | 2020226696 A1 | 12-11-2020 |
| WO 2022094629 | A1 | 05-05-2022 | CN | 116547727 A | 04-08-2023 |
| | | | EP | 4238069 A1 | 06-09-2023 |
| | | | JP | 2023548347 A | 16-11-2023 |
| | | | KR | 20230097127 A | 30-06-2023 |
| | | | US | 2024079138 A1 | 07-03-2024 |
| | | | WO | 2022094629 A1 | 05-05-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ANEGONDI et al.** Deep Learning to Predict Geographic Atrophy Area and Growth Rate from Multimodal Imaging. American Academy of Ophtalmology, 2022
- **THULASIDASAN et al.** On mixup training: Improved calibration and predictive uncertainty for deep neural networks. *Neural Information Processing Systems*, 2019
- **VERMA et al.** Manifold mixup: Better representations by interpolating hidden states. *International Conference on Machine Learning*, 2018
- **CHEN et al.** *Neural Ordinary Differential Equations*, 2018
- **CHEN et al.** *Neural Ordinary Differential Equations*, 2018, https://doi.org/10.48550/ARXIV.1806.07366
- **BAYTAS et al.** *Patient subtyping via time-aware lstm networks*, 2017, https://doi.org/10.11.15/3097983.3097997Time-LSTM